(19) Europäisches Patentamt
European Patent Office
Office européen des brevets

(11) Publication number: 0 369 776 B1

# EUROPEAN PATENT SPECIFICATION

(45) Date of publication of patent specification: 24.05.95

(51) Int. Cl.6: **A61K 33/24**, A61K 31/72, A61K 31/70, A61K 35/72, A61K 35/74, A61K 35/76, A61K 35/84

(21) Application number: 89311840.6

(22) Date of filing: 15.11.89

The file contains technical information submitted after the application was filed and not included in this specification

(54) **Use of a composition containing organic germanium for the manufacture of a medicament for treating AIDS.**

(30) Priority: 15.11.88 JP 286810/88
27.11.88 JP 299202/88

(43) Date of publication of application:
23.05.90 Bulletin 90/21

(45) Publication of the grant of the patent:
24.05.95 Bulletin 95/21

(84) Designated Contracting States:
CH DE ES FR GB IT LI

(56) References cited:
EP-A- 0 016 444
EP-A- 0 235 906
FR-A- 2 418 805

PATENTS ABSTRACTS OF JAPAN, vol. 13, no. 151 (C-584)(3499), 12th April 1989; & JP-A-63 307 821 (ASAI GERUMANIUMU KEN-KYUSHO K.K.) 15-12-1988

(73) Proprietor: SANWA KAGAKU KENKYUSHO CO., LTD.
No. 35, Higashi-sotobori-cho
Higashi-ku
Nagoya-shi
Aichi-ken (JP)

(72) Inventor: Sawai, Kiichi
36-14, Ninomiya 1-chome
Funabashi-shi
Chiba-ken (JP)
Inventor: Kurono, Masayasu
6-7, Sasaonishi 3-chome
Touincho
Inabegun
Mie-ken (JP)
Inventor: Asano, Kyoichi
39-95, Moriyama-Kitayama
Moriyama-ku
Nagoya-shi
Aichi-ken (JP)

MEDICAL HYPOTHESES, vol. 26, no. 3, July 1988, pages 207-215, Longman Group UK LTD; S. GOODMAN: "Therapeutic effects of organic Germanium"

PATENT ABSTRACTS OF JAPAN, vol. 10, no. 43 (C-329)(2100), 20th February 1986; & JP-A-60 190 714 (SANWA KAGAKU KENKYUSHO K.K.) 28-09-1985

INT. J. IMMUNOTHERAPHY, vol. 3, no. 2, 1987, pages 97-103; S. ARAI et al.: "Induction of interferon production by natural killer cells by an organogermanium compound, Ge132"

Inventor: **Mitani, Takahiko**
**881-3, Ageki**
**Hokuseicho-oaza**
**Inabe-gun (JP)**
Inventor: **Ninomiya, Naohisa**
**5-79, Motoyagoto**
**Tenpaku-ku**
**Nagoyashi**
**Aichi-ken (JP)**
Inventor: **Ishiwata, Yoshiro**
**8-31, Ikeshita daini-kodan**
**916 Kakuousan-dori**
**Chigusa-ku**
**Nagoya-shi**
**Aichi-ken (JP)**
Inventor: **Yokochi, Syoji**
**564-22, Onaka-shinden-oaza**
**Kuwana-shi**
**Mie-ken (JP)**
Inventor: **Yamada, Kaneo**
**10-21, Kita-Aoyama 2-chome**
**Monato-ku**
**Tokyo (JP)**
Inventor: **Taki, Masashi**
**2098-4, Kami-Turama**
**Sagamihara-shi**
**Kanagawa-ken (JP)**
Inventor: **Meguro, Takashi**
**1222-15, Tana**
**Sagamihara-shi**
**Kanagawa-ken (JP)**
Inventor: **Minamitani, Mikio, Sanwa Kagaku Kenkyusho Co Ltd**
**Higashi-sotobori-cho 35,**
**Higashi-ku**
**Nagoya-shi,**
**Aichi-ken (JP)**
Inventor: **Matumoto, Takao**
**15-5-802, Nishi-shinjuku 3-chome**
**Shinkuku-ku**
**Tokyo (JP)**
Inventor: **Shiokawa, Yuichi**
**37-14, Sengoku 4-chome**
**Bunkyoku**
**Tokyo (JP)**

(74) Representative: **Diamond, Bryan Clive et al**
**Gee & Co.**
**Chancery House**
**Chancery Lane**
**London WC2A 1OU (GB)**

## Description

The present invention relates to the use of a composition of an organic germanium compound for treating the acquired immune deficiency syndrome ("AIDS").

Various compounds have been tested as inducers of interferon, and some are now available, e.g. under the trade names Picibanil, Krestin and Maruyama vaccine. Additionally there are known carcinostatics (JP-A-53-36662) and virucides (JP-A-55-8730) containing as effective ingredients dead bacterial mixtures of Pneumococcus, hemolytic Streptococcus, Staphylococcus, Neisseria catarrhalis, Tetracoccus, Pseudomonas aeruginosa Klebsiella and Haemophilus influenzae.

AIDS is a disease caused by human immuno-deficiency viruses (hereinafter HIV for short). When infected with such viruses, helper T cells of lymphocytes are destroyed, to induce cellular immuno-deficiency and eventually opportunistic infection, Kaposi's sarcoma, etc., thus HIV causes high mortality rates.

When compared with human hepatitis virus infection, human leukemic retrovirus infection and other virus infections, such AIDS virus infections (virus carriers) can be much higher in their incidence and in their subsequent mortality. Moreover, medicines now available against AIDS are mainly inhibitor systems for the synthesis of nucleic acid, which have a grave disadvantage of attacking normal tissues rather than producing a virucidal action. Thus, there is a need for medicines of greater safety in use and having a high cure rate against AIDS. JP-A-53-21992 discloses that germanium polymers are effective for curing psychoses, neuroses, cacochymia, cardiopathy, angiopathy, digestive system dysfunctions, dermopathy, allergosis and renal and hepatic dysfunctions, as well as various diseases relating to obstetrics and gynaecology and pediatrics. Additionally, JP-A-54-75177 and other publications teach that germanium polymers have an anti-DNA viral action upon influenza, Variola crystallina (herpes) and other viral infections.

S. Goodman, in "Medical Hypotheses" (July 1988), UK, Vol 26, pages 207-215, describes the therapeutic effects of various germanium compounds, e.g. carboxyethyl germanium sesquioxide (Ge-132) and an azaspirine germanium compound, and describes their antitumor and immunological known properties and suggests that they might be effective in the treatment and/or prevention of AIDS.

There is no description therein of any such treatment being carried out, nor of the formula for the range of germanium oxide compounds used in this invention.

As is well known, an organic germanium compound may be prepared as follows. Trichlorogermanium is allowed to react with acrylonitrile to obtain trichlorogermanium ethylnitrile. This is then hydrolyzed with an acid into trichlorogermanium ethyl carboxylate. Thereafter, this carboxylate is chlorinated with thionyl chloride into trichlorogermanium ethyl chloride which, when exposed to water or aqueous ammonia, gives bis-$\beta$-ethyl carboxylic acid amide germanium sesquioxide. The thus obtained organic germanium compound is not only different in structure and physical properties from the compound used in the present invention, but has the grave defect of differing in action from lot to lot; and its action and behaviour are uncertain.

To overcome the disadvantages of these known organic germanium compounds, a germanium polymer compound is disclosed in JP-A-54-115324. This compound is essentially similar in structure to the substance used in the present invention and produces a strong effect in relatively small dosages, and is thus said to be a useful compound that satisfies various requirements for medicines. However, problems with it are that it is not only apt to undergo chemical changes during storage over an extended period of time, but is not easily absorbed into the human body in a stable manner, when administered thereto. Consequently, this Ge-132 germanium compound is now administered at relatively large doses, to overcome its decomposition but because germanium is a rare element which should be used sparingly, there is a need for preparations and compositions in which the germanium compound can directly exert a strong action when used in small amounts. The induction of interferon production by natural killer cells by Ge-132 is disclosed in Int. J. Immunotherapy, III(2), pp. 97-103 (1987).

Our JP-A-59-44066 discloses that the pharmacological action of the organic germanium compound used in the present invention can be enhanced by the addition of a carrier for pharmaceutical preparations such as saccharides (e.g., lactose) or high-molecular compounds (e.g., proteins); we have thus provided a medicinal composition for external application for the purpose of preventing urticaria and like conditions due to immunodeficiency; we have now found that the same organic germanium compound also has a second medical use, an anti-retroviral action, inter alia, an effect on making AIDS asymptomatic.

The composition used in the present invention contains (a) as a primary component an organic germanium compound expressed by the following formula:

$( \overset{>}{\phantom{.}} GeCH_2CH_2COOH)_nO_{1.5n}$

wherein $n$ is an integer of 1 or 2 or more, in the form of white acicular or needle crystals, having a solubility of 1.57g/100 ml in water at 25°C and a melting (decomposition) point of 240°C, and (b) an activating carrier selected from interferon producers, chondroitin sulfate, chitin sulfate and dextran sulfate. We provide as a second medical use, the use of said composition for the manufacture of a medicament for the treatment of AIDS.

An interferon producer used is preferably a live or dead bacterial component selected from polynucleotides, influenza viruses, Haemophilus influenza, tubercle bacillus, hemolytic Streptococcus and Basidiomycetes or yeast cell polysaccharide. However, it is desired that the use of live preparations is avoided as much as possible for patients attacked by AIDS. For such patients, the dead bacterial component or a composition of the germanium compound with a polymeric compound, saccharide or the like should preferably be used.

As a saccharide, lactose is preferred, preferably added in an amount of 50 to 80 % by weight such that it has no influence upon the pharmaceutical action of the interferon inducer. This is also true of other customary vehicles. There is thus obtained a stable, action-enhancing composition.

Suitable polymeric carriers include gelatin, pepsin, serum albumin, globulin, protamine, cellulosic carriers, vinylic carriers, acrylic polymer carriers, peptone, polypeptone, yeast extracts, tryptone, tryptose, and dextrose.

Thus, the germanium compounds used in the invention take part in the enhancement of production of anti-HIV substances such as interferon in living bodies and so have a strong antiviral action therein. They are also very effective for making AIDS asymptomatic or curing AIDS.

The compositions used in the present invention are of high stability so that they can be continuously administered and used with stable pharmacological action.

Furthermore the compositions used have an advantage of being orally administered and thus easier to use than other drugs.

In the drawings (not illustrative of the invention):

Figure 1 is a graph of the results of experiments on the effect of the present composition upon the enhancers of interferon biosynthesis of mice infected with influenza viruses, and

Figure 2 is a graph of the results of experiments on the effect of the present composition upon the enhancement of the production of interferon of BCG-infected mice.

In the drawings, one asterisk * means P 0.05, ** P 0.01 and *** P 0.001 where P is probability.


EXAMPLES

The present invention will now be illustrated by the following examples.

Preparation of Organic Germanium Compound

In 2 l of ethyl alcohol dissolved 252 g (1 mole) of 3-trichlorogermanium propionic acid, and 1.3 l of water are added under agitation to the solution. After allowing to stand for one day, crystals are obtained by suction filtration, washed with acetone and dried under reduced pressure.

As a result, the desired organic germanium compound was obtained in the form of white acicular crystals.

Yield: 90%

Melting (Decomposition) Point: 240°C.

Solubility at 25°C in Water: 1.57 g/100 m .

Pharmaceutical and Pharmacological Effects

The composition used in Examples 1 and 2 was obtained by adding and mixing an organic germanium compound with 1% aqueous solution of pepsin at a concentration of 1%, followed by freeze drying.

1. Action of the Present Composition upon the Inducement of Interferon by Influenza Viruses

(i) Experimental Procedures

A group of five BALB/C mice were endotracheally infected with influenza viruses (A/PR/8 strains) at a dose of 8.7 PFU/mouse. Just after the infection, the composition of the present invention was orally administered to the mice at doses of 0.1 mg, 1.0 mg, 10.0 mg and 100.0 mg, calculated as organic germanium (the primary ingredient). After three days of the infection with influenza viruses, the lungs were removed from the mice, homogenized and centrifuged at 20000 G to obtain a supernatant. The interferon activity of the supernatant was determined in terms of L929 cytopathic inhibition by vesicular stomatitis viruses (VSV).

(ii) Experimental Results

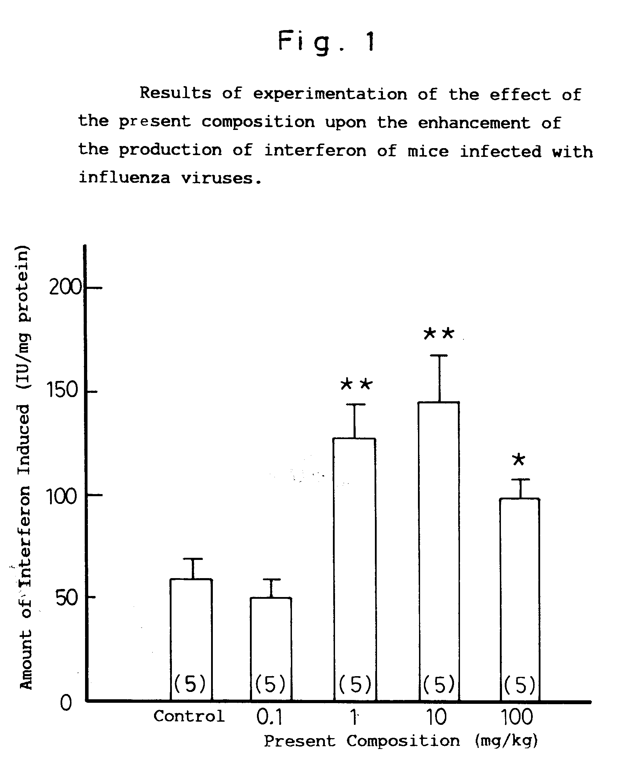

The results of the experiments are presented in Figure 1, from which it is noted that the composition of the present invention increases the production of interferon by a factor of 2.2, 2.5 and 1.7 at the respective doses of 1.0 mg/kg, 10.0 mg/kg and 100.0 mg/kg, calculated as organic germanium which is the primary ingredient.

(iii) Determination of the Type of Interferon Produced

The serum of a mouse infected with influenza viruses and administered with the present present composition was treated at $4\,°C$ for 1 hour with an anti-mouse $\alpha/\beta$-interferon antibody, an anti-mouse $\beta$-interferon antibody and an anti-mouse $\gamma$-interferon antibody to determine the activity of interferon. In consequence, it was found that the interferon activity of the serum of the mouse under examination disappeared by treatment with the anti-mouse $\alpha/\beta$-interferon antibody, but it remained intact when treated with the anti-mouse $\beta$-interferon antibody and anti-mouse $\gamma$-interferon antibody. If therefore it appears that the interferon of the mouse infected with influenza viruses and enhanced by the present composition in terms of interferon productivity was an $\alpha$-type.

2. Enhancement of Interferon Productivity of BBC Vaccine-Sensitized Mouse.

(i) Experimental Procedures

A group of seven C57/BL/6 mice were used. Dry BCG vaccine suspended in 0.9 % physiological saline was injected into the tail veins of the mice at a dose of 0.25 mg/mouse for sensitization. After ten days of the sensitization, they were orally administered with the composition of the invention at dosages of 0.1, 1, 10 and 100 mg/kg calculated as organic germanium. On the day following the final administration (14 days after sensitization), BCG was intravenously injected at a dose of 0.1 mg/mouse. Three hours later, blood was collected to determine the interferon activity of the separated serum.

(ii) Results of Experimentation

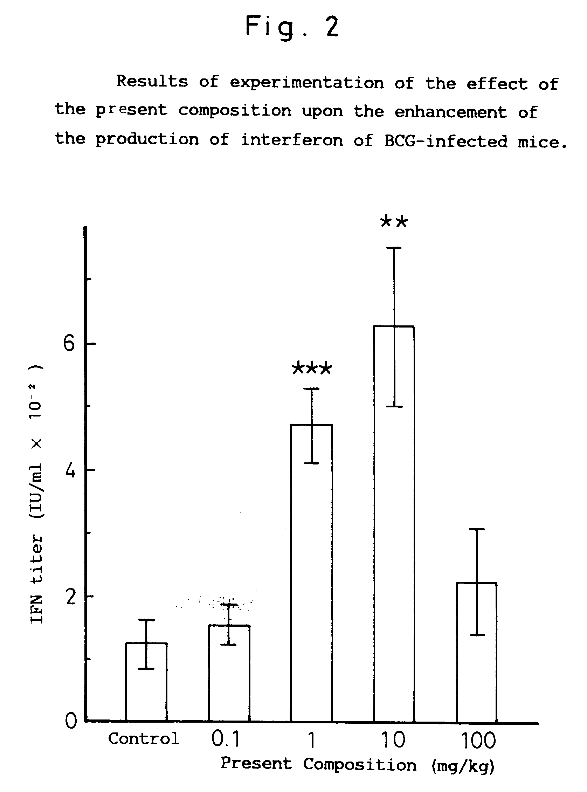

The experimental results are graphically illustrated in Figure 2, from which it is found that the composition according to the present invention signicantly enhances the interferon production of the BCG-sensitized mouse and increases the production of interferon by a factor of 3.7 and 5.0 at the respective doses of 1.0 mg/kg and 10.0 mg/kg, calculated as organic germanium, over a control group.

(iii) Determination of the Type of Interferon of BCG-Sensitized Mouse Enhanced in Terms of its Production by the Composition for use in the invention

The serum of a BCG-sensitized mouse administered with the present composition was dialyzed with 0.1 mg of a lysin-HC1 buffer of pH 2.0 for 24 hours, to determine its interferon activity.

In consequence, the interferon of the serum of the BCG-sensitized mouse administered with the present composition was deactivated. On the other hand, standard $\alpha/\beta$-interferon treated in a similar manner as mentioned above showed only a 22 % decrease of activity.

BCG-sensitized mice are known to produce $\gamma$-interferon. Thus, it was revealed that the interferon of the BCG-sensitized mouse enhanced in terms of its production was a $\gamma$-type.

3. Stabilization of Commercially Available Preparations

Tests were made of the effect of the present composition upon the enhancement of the inducement of interferon in commercially available preparations. As an interferon inducer, use was made of an antiviral preparation containing as an effective ingredient different types of dead bacteria (Broncasma berna JP-A-62-34725).

(i) Experimental Procedures

A group of five BALB/C mice were intraperitoneally administered with 5 ml/kg of Broncasma berna, immediately after which the present composition was orally administered thereto at a dose of 1.0 mg/kg calculated as organic germanium. After 20 hours of administration, blood was gathered from each mouse to determine the interferon activity of the separated serum.

(ii) Results of Experimentation

It was evident that, as shown in Table 1, the present composition successfully increases the inducement of interferon of Broncasma berna by a factor of about 3.

Table 1

|  | Interferon Activity (IU/ml) |
| --- | --- |
| Control | not detected |
| Broncasma berna | 110 |
| Broncasma berna + Present Composition | 320 |

4. Results of Clinical Tests

Six HIV-infected subjects, four positive and two negative (all being male haemophiliacs between the ages of 6 and 22, asymptomatic carriers) were administered the preparation of the present invention (to be described later) over a period of seven months (at a dose of 60 mg/day calculated as the organic germanium compound, but at a dose of 40 mg/day for under ten years of age) to make immunological studies of the number of lymphocytes, OKT4 and OKT4/8. In the meantime, virus markers such as HIV, HIV antigens and HIV antibodies were measured with clinical observations. There was no significant difference in the number of lymphocytes, say, $2470\pm311/\mu l$ before administration and $2349\pm312/\mu l$ after six months of administration. The number of OKT4 increased from $463\pm64/\mu l$ before administration to $549\pm89/\mu l$ after two months of administration and $629\pm97/\mu l$ after six months of administration. The number of OKT8 decreased slightly from $1303\pm174/\mu l$ before administration to $1154\pm153/\mu l$ after six months of administration.

The number of OKT4/8 showed a gradually increasing tendency, say, from $0.36\pm0.03$ before administration to $0.42\pm0.02$ after one month of administration and $0.51\pm0.05$ after six months of administration.

The separation of viruses in the blood was carried out by the CDC (Center for Disease Control), while HIV antigens were measured on a daily basis by the Western Blotting method P24.

Of the four HIV-positive subjects, two underwent no change after the six-month administration of the present compound, but another two subjects (A and B) showed no sign of HIV at all (see Table 2).

On the other hand, there was no clinically noticeable side effect, and two subjects had a good appetite and gained weight.

## Table 2

### Changes of Virus Markers of HIV-Positive Subjects

### (Results Obtained as of October, 1988)

|              |              | 0 week | 6 months | 7 months |
|--------------|--------------|:------:|:--------:|:--------:|
|              | HIV Separation | +    | −        | −        |
| Subject A    | HIV Antigen    | −    | −        | −        |
|              | HIV Antibody   | +    | +        | +        |

|              |              | − 5 weeks | 0 week | 2 weeks | 6 months | 7 months |
|--------------|--------------|:---------:|:------:|:-------:|:--------:|:--------:|
|              | HIV Separation | +       | +      | +       | −        | −        |
| Subject B    | HIV Antigen    | +       | +      | +       | +        | +        |
|              | HIV Antibody   | +       | +      | +       | +        | +        |

In two subjects, each diagnosed as HIV-positive as of October, 1988, the HIV was killed (subject became HIV-negative) after 11 and 12 months of the initial administration. This effective action encouraged an additional four subjects to receive treatment; in two of whom the HIV was killed after 4 and 7 months after administration. Later, the number of subjects being treated increased and reached twelve as of September, 1989.

Referring to the first-mentioned four subjects, HIV was killed after 6 months at the earliest and 12 months at the latest. From the results of the later tests, it was also noted that HIV was killed after 4 months of administration.

Thus, it was shown that the preparations of the present invention provide more effective drugs for making AIDS asymptomatic and curing AIDS.

5. Examples of Pharmaceutical Preparations

(i) Nebulizer

| | |
|---|---|
| Broncasma berna | 10 ml |
| Solution obtained by adding and dissolving the present organic germanium compound into a 4 % solution of serum albumin at a concentration of 1.0 % | 90 ml |

The above ingredients are mixed together into a nebulizer preparation.

(ii) Granules

| | |
|---|---|
| Krestin (Protein-bonded polysaccharide) | 1 g |
| Present organic germanium compound | 20 mg |
| Hydroxypropyl cellulose | 75 mg |
| Magnesium stearate | 7.5 mg |
| Lactose | suitable quantity |
| | 1.5g per chartula (folded paper) |

The organic germanium compound and lactose are mixed and melted together under wet conditions, followed by drying and granulation. The product is mixed with the other ingredients to form granules in a customary manner.

(iii) Capsules 1

| Krestin | 300 mg |
|---|---|
| Present organic germanium compound | 10 mg |
| Hydroxypropyl cellulose | 15 mg |
| Magnesium stearate | 3 mg |
| Lactose | suitable quantity |
| | 380 mg per capsule |

(iv) Capsules 2

| Present organic germanium compound | 10 mg |
|---|---|
| Lactose | 165.5 mg |
| (Hydroxypropyl cellulose | 2.7 mg |
| Magnesium stearate | 1.8 mg |
| | 180 mg per capsule |

For each type of capsules, the organic germanium compound and lactose are mixed and melted together under wet conditions, followed by drying and granulation, and the product is mixed with the other ingredients and encapulated in customary manner.

6. Example of Preparation Used Exclusively with Interferon Inducer

Tablets

| Composition obtained by adding and dissolving the organic germanium compound into an 1 % aqueous solution of pepsin at a concentrate of 1 %, followed by freeze-drying | 60 mg |
|---|---|
| Carboxymethyl cellulose calcium | 7 mg |
| Light silicic anhydride | 1 mg |
| Magnesium stearate | 7 mg |
| Lactose | suitable quantity |
| | 165 mg per tablet |

This preparation is to be administered orally in combination with an interferon inducer which is administered by injection.

## Claims

**1.** The use of a composition containing (a) as a primary ingredient an organic germanium compound which is expressed by the following rational formula:

$$( \overset{\geq}{} GeCH_2CH_2COOH)_nO_{1.5n}$$

wherein $\underline{n}$ is an integer of 1 or 2 or more, which compound is in the form of white needle crystals, has a solubility of 1.57g/100 ml in water at 25 °C and a melting (decomposition) point of 240 °C, and (b) an activating carrier selected from interferon producers, chondroitin sulfate, chitin sulfate and dextran

8

sulfate,

for the manufacture of a medicament for the treatment of the acquired immune deficiency syndrome.

2. Use as claimed in Claim 1, wherein the composition is in a form suitable to be orally administered.

**Patentansprüche**

1. Verwendung einer Zusammensetzung enthaltend

(a) als primären Bestandteil eine organische Germaniumverbindung, die durch die folgende rationale Formel ausgedrückt wird:

$$(\rightarrow GeCH_2 CH_2 COOH)_n O_{1,5n}$$

worin n eine ganze Zahl von 1 oder 2 oder mehr ist, wobei diese Verbindung in Form von weißen Nadelkristallen vorliegt, eine Löslichkeit von 1,57 g/100 ml in Wasser bei 25°C und einen Schmelzpunkt (Zersetzungspunkt) von 240°C hat, und
(b) einen aktivierenden Träger, ausgewählt aus Interferonproduzenten, Chondroitinsulfat, Chitinsulfat und Dextransulfat,
zur Herstellung eines Arzneimittels zur Behandlung des erworbenen Immundefizienzsyndroms.

2. Verwendung nach Anspruch 1, worin die Zusammensetzung in einer Form vorliegt, die zur oralen Verabreichung geeignet ist.

**Revendications**

1. Utilisation d'une composition contenant (a) comme ingrédient primaire, un composé organique du germanium qui est exprimé par la formule rationnelle suivante :

$$(\rightarrow GeCH_2 CH_2 COOH)_n O_{1,5n}$$

(dans laquelle n est un nombre valant 1 ou 2 ou davantage), ce composé étant sous la forme de cristaux aciculaires blancs, qui ont une solubilité de 1,57 g/100 ml dans de l'eau à 25°C et un point de fusion (avec décomposition) de 240°C et (b) un support, véhicule ou excipient activateur, choisi parmi les producteurs d'interféron, du sulfate de chondronitine, du sulfate de chitine et du sulfate de dextrane, pour la fabrication d'un médicament destiné à traiter le syndrome de l'immunodéficience acquise.

2. Utilisation selon la revendication 1, dans laquelle la composition est sous une forme convenant pour de l'administration par voie orale.

# Fig. 1

Results of experimentation of the effect of the present composition upon the enhancement of the production of interferon of mice infected with influenza viruses.

# Fig. 2

Results of experimentation of the effect of
the present composition upon the enhancement of
the production of interferon of BCG-infected mice.